# EUROPEAN PATENT APPLICATION

(11) **EP 4 578 527 A1**
(43) Date of publication of application: **02.07.2025**
(21) Application number: 24167130.4
(22) Date of filing: 28.03.2024
(51) Int. Cl.: B01D 3/14, B01D 19/00, B01D 61/36, C07D 207/267

(54) **HIGH-PURITY NMP RECOVERY DEVICE AND RECOVERY PROCESS COMPRISING A DEOXYGENATING DEVICE**

(30) Priority: 29.12.2023 CN 202311863092
(71) Applicant: Guangdong Tian Rui De New Energy Technology Co., Ltd., Shenzhen 518000 (CN)
(72) Inventor: YAN, Yongjun, Shenzhen (CN); LI, Tianyou, Shenzhen (CN); MENG, Jili, Shenzhen (CN); ZHU, Chunfang, Shenzhen (CN)
(74) Representative: Zaboliene, Reda

(57) **Abstract**

The present invention discloses a high-purity NMP recovery device and recovery process. The high-purity NMP recovery device includes a deoxygenation and dehydration apparatus. The deoxygenation and dehydration apparatus includes a deoxygenation preheater, a first deoxygenator, a dehydration tower, a dehydration tower-top condenser, a dehydration tower-top reflux tank, a molecular sieve membrane device raw material tank, a molecular sieve membrane pipe feed pump, a preheater located before a molecular sieve membrane, a NaA molecular sieve membrane component, a second deoxygenator, and a dehydration tank located after a molecular sieve membrane. The deoxygenation preheater is connected to a raw material feed pipe and the first deoxygenator, and the dehydration tower is connected to the first deoxygenator, the dehydration tower-top condenser, and the molecular sieve membrane device raw material tank. The dehydration tower-top reflux tank is connected to the dehydration tower-top condenser and the dehydration tower. The molecular sieve membrane pipe feed pump is connected to the molecular sieve membrane device raw material tank and the preheater located before a molecular sieve membrane. The NaA molecular sieve membrane component is connected to the preheater located before a molecular sieve membrane, the molecular sieve membrane device raw material tank, and the second deoxygenator. An NMP product obtained after treatment by the high-purity NMP recovery device almost has no by-products.

## Description

### TECHNICAL FIELD

The present invention relates to the field of NMP recovery and extraction technologies, and in particular, to a high-purity NMP recovery device and recovery process.

### BACKGROUND

1-Methyl-2-pyrrolidone (NMP) is an important chemical solvent with low toxicity, high boiling point, a strong dissolving capability, and good selectivity and stability. 1-Methyl-2-pyrrolidone (NMP) is widely used in lithium battery manufacturing. NMP is stable in a neutral condition, but is prone to ring opening to form acid and other products in an acid or alkaline condition, and oxygen, water, and temperature have impact on decomposition of NMP.

Currently, there are many methods for NMP recovery in the lithium battery industry. One method is distillation for a liquid mixture containing NMP, and this method can effectively remove impurities such as water because the boiling point of NMP is high. Another method is to obtain high-purity NMP through extraction, because NMP is highly polar and insoluble in a nonpolar solvent, but soluble in a polar solvent (for example, trichloromethane). In addition, there are also processes such as molecular sieve membrane vaporization and infiltration. However, the foregoing methods cause hydrolysis and oxidation of different quantities of NMP during use. Main products of the hydrolysis and oxidation of NMP include N-Methylsuccinimide (NMS), and a small quantity of N-Methylacetamide, 2-Pyrrolidone, and 1,3-Cyclopentanedione. These products pollute an NMP product, and consequently, purification of NMP does not satisfy a purity requirement of electronic-grade NMP, and subsequent refining treatment is needed, or these products pollute a molecular sieve membrane in application of the molecular sieve membrane, which shortens an operation cycle of the molecular sieve membrane, and shutdown for maintenance is needed.

Although nitrogen introduced into an NMP recovery system can play a protective role, a large quantity of by-products are produced. One reason is as follows: The NMP solution still has some dissolved oxygen, and a side reaction still occurs under a heating condition. Another reason is as follows: Currently, a controlled temperature in NMP purification is high, and when the temperature is 80 °C, the NMP aqueous solution turns yellow and a hydrolysis reaction occurs. At present, in an industrial production procedure, a temperature for NMP recovery is generally higher than 100 °C, and a product obtained after vacuum distillation still contains a small quantity of N-Methylsuccinimide (NMS) and N-Methylacetamide. In addition, oxidative decomposition of NMP depends on a time in which NMP stays in a high-temperature state, and a shorter staying time indicates a lower degree of the oxidative decomposition.

In conclusion, it is difficult for an existing NMP recovery device to produce high-purity NMP.

### SUMMARY

A technical issue to be resolved in the present invention is to provide a high-purity NMP recovery device and a recovery process that is based on the high-purity NMP recovery device.

To resolve the foregoing technical issue, technical solution 1 used in the present invention is as follows: A high-purity NMP recovery device is provided, including a deoxygenation and dehydration apparatus, where the deoxygenation and dehydration apparatus includes a deoxygenation preheater, a first deoxygenator, a dehydration tower, a dehydration tower-top condenser, a dehydration tower-top reflux tank, a molecular sieve membrane device raw material tank, a molecular sieve membrane pipe feed pump, a preheater located before a molecular sieve membrane, a NaA molecular sieve membrane component, a second deoxygenator, and a dehydration tank located after a molecular sieve membrane; the deoxygenation preheater is connected to a raw material feed pipe and the first deoxygenator, and the dehydration tower is connected to the first deoxygenator, the dehydration tower-top condenser, and the molecular sieve membrane device raw material tank; the dehydration tower-top reflux tank is connected to the dehydration tower-top condenser and the dehydration tower; the molecular sieve membrane pipe feed pump is connected to the molecular sieve membrane device raw material tank and the preheater located before a molecular sieve membrane; and the NaA molecular sieve membrane component is connected to the preheater located before a molecular sieve membrane, the molecular sieve membrane device raw material tank, and the second deoxygenator.

To resolve the foregoing technical issue, technical solution 2 used in the present invention is as follows: A high-purity NMP recovery process is provided, where the process is based on the foregoing high-purity NMP recovery device.

Beneficial effects of the present invention are as follows: The high-purity NMP recovery device can implement pretreatment for deoxygenation of raw material liquid, low-temperature treatment for dehydration, and refining of a short staying time at high temperature after dehydration, and an NMP product obtained after treatment almost has no by-products, so that the device can be used to produce high-purity electronic-grade NMP.

Low-temperature dehydration and deoxygenation: deoxygenation at a temperature of 50 °C to 55 °C, dehydration in the dehydration tower at a temperature lower than 80 °C, and purification in the NaA molecular sieve membrane component at a temperature of 80 °C to 95 °C.

Reduction in a staying time during high-temperature refining: A staying time is approximately 5 seconds to 10 seconds when the scraper falling-film evaporation reboiler is used, an unvaporized material at an outlet of the reboiler is cooled to 90 °C, a staying time in a cooling procedure is controlled to be approximately 10 seconds to 20 seconds, and a total staying time is controlled to be within 30 seconds. This reduces oxidative decomposition of NMP to a greater extent, and high-purity electronic-grade NMP can be obtained.

Low-temperature dehydration and deoxygenation in the high-purity NMP recovery device effectively reduce frequent maintenance of the NaA molecular sieve membrane component due to pollution by small molecules obtained after decomposition of NMP, resolve, from the source, a problem of troublesome maintenance caused by oxidation and decomposition of NMP, greatly prolong a service life and maintenance cycle of the NaA molecular sieve membrane component, and help improve production efficiency.

For some materials that contain only water or production of an industrial-grade NMP product, only the deoxygenation and dehydration apparatus in the NMP recovery device is needed without using a refining apparatus. It can be learned that the NMP recovery device has an advantage of high flexibility of use.

### BRIEF DESCRIPTION OF DRAWINGS

To describe technical solutions in embodiments of the present invention or in a conventional technology more clearly, the following briefly describes accompanying drawings that are required for descriptions of embodiments or the conventional technology. It is clear that the accompanying drawings in the following descriptions show merely some embodiments of the present invention, and a person of ordinary skill in the art may still derive other drawings from structures shown in these accompanying drawings without creative efforts.
FIG. 1 is a simplified schematic diagram of a structure of a deoxygenation and dehydration apparatus in a high-purity NMP recovery device according to Embodiment 1 of the present invention;
FIG. 2 is a simplified schematic diagram of a structure of a refining apparatus in a high-purity NMP recovery device according to Embodiment 1 of the present invention;
FIG. 3 is a schematic diagram of a structure of a first deoxygenator in a high-purity NMP recovery device according to Embodiment 1 of the present invention; and
FIG. 4 is a schematic diagram of a structure of a first deoxygenator of another structure in a high-purity NMP recovery device according to Embodiment 1 of the present invention.

### Reference numerals:

11: deoxygenation preheater; 12: dehydration tower thermosiphon reboiler; 13: dehydration tower-top condenser; 14: dehydration tower-top vacuum condenser; 15: preheater located before a molecular sieve membrane; 16: cooler located after a molecular sieve membrane; 17: vacuum condenser located after a molecular sieve membrane; 18: refining tower scraper falling-film evaporation reboiler; 19: refining tower feed-before preheater; 110: refining tower kettle discharge cooler; 111: refining tower-top condenser; 112: refining tower-top vacuum condenser; 21: deoxygenator located before a dehydration tower; 22: deoxygenator located after a dehydration tower; 23: deoxygenator located before a refining tower; 31: dehydration tower feed pump; 32: dehydration tower kettle discharge pump; 33: dehydration tower-top reflux discharge pump; 34: molecular sieve membrane pipe feed pump; 35: molecular sieve membrane pipe dehydration discharge pump; 36: refining tower feed pump; 37: refining tower return and tower kettle discharge pump; 38: refining tower-top reflux discharge pump; 39: NMP product discharge pump; 41: dehydration tower; 42: refining tower; 51: dehydration tower-top reflux tank; 52: molecular sieve membrane device raw material tank; 53: dehydration tank located after a molecular sieve membrane; 54: refining tower raw material tank; 55: refining tower kettle liquid temporary storage tank; 56: refining tower-top reflux tank; 57: NMP product cooling tank; 6: NaA molecular sieve membrane component; 71: box; 711: solvent inlet; 712: solvent outlet; 72: partition plate; 721: first vertical section; 722: inclined section; 723: second vertical section; 73: chamber; 731: gas-phase space; 732: first space; 733: second space; 74: vacuum pumping hole; 75: gas introducing opening; 76: overflow weir; 77: aeration apparatus; 78: liquid outlet; 79: gas-liquid mixing pump; 710: main pipe; 7101: inert gas access pipe;
S1: first pipe; S2: second pipe; S3: third pipe; S4: fourth pipe; S5: fifth pipe; S6: sixth pipe; S7: seventh pipe; S8: eighth pipe; S9: ninth pipe; S10: tenth pipe; S11: eleventh pipe; S12: twelfth pipe; S13: thirteenth pipe; S14: fourteenth pipe; S15: fifteenth pipe; S16: sixteenth pipe; S17: seventeenth pipe; S18: eighteenth pipe; S19: nineteenth pipe; S20: twentieth pipe; S21: twenty-first pipe; S22: twenty-second pipe; S23: twenty-third pipe; S24: twenty-fourth pipe; S25: twenty-fifth pipe; S26: twenty-sixth pipe; S27: twenty-seventh pipe; S28: twenty-eighth pipe; S29: twenty-ninth pipe; S30: thirtieth pipe; S31: thirty-first pipe; S32: thirty-second pipe; S33: thirty-third pipe; S34: thirty-fourth pipe; S35: thirty-fifth pipe; S36: thirty-sixth pipe; S37: thirty-seventh pipe; S38: thirty-eighth pipe; S39: thirty-ninth pipe.

### DESCRIPTION OF EMBODIMENTS

Achievement of objectives, functional features, and advantages of the present invention are further described with reference to embodiments and accompanying drawings.

The following clearly and completely describes technical solutions in embodiments of the present invention with reference to the accompanying drawings in embodiments of the present invention. It is clear that the described embodiments are merely some but not all of embodiments of the present invention. All other embodiments obtained by a person of ordinary skill in the art based on embodiments of the present invention without creative efforts shall fall within the protection scope of the present invention.

It should be noted that, if embodiments of the present invention involve directional indications such as above, below, left, right, front, and back, the directional indications are merely used to explain relative position relationships, movement conditions, or the like of components in a specific attitude, as shown in the accompanying drawings. If the specific attitude changes, the directional indications change accordingly.

In addition, if there are descriptions involving "first", "second", and the like in embodiments of the present invention, the descriptions of "first", "second", and the like are merely intended for an objective of description, and shall not be understood as an indication or implication of relative importance or implicit indication of a quantity of indicated technical features. Therefore, a feature defined by "first" or "second" may explicitly or implicitly include at least one feature.

In addition, "and/or" in this specification means that three parallel solutions are included. For example, "A and/or B" indicates the following three solutions: A is included, B is included, or both A and B are included. In addition, the technical solutions in various embodiments can be combined with each other, which should be based on implementation by a person of ordinary skill in the art. When the combination of technical solutions is contradictory or cannot be implemented, it should be considered that such a combination of technical solutions does not exist, and is not within the protection scope claimed by the present invention.

In this application, unless otherwise specified and limited, terms such as "mount", "link", "connect", and "fasten" should be understood broadly. For example, the term "connect" may indicate a fixed connection, a detachable connection, or integration; may indicate a mechanical connection or an electrical connection; may indicate a direct connection or an indirect connection implemented by using an intermediate medium; or may indicate communication inside two elements or an interaction relationship between two elements. A person of ordinary skill in the art may interpret specific meanings of the foregoing terms in this application according to specific cases.

### Embodiment 1

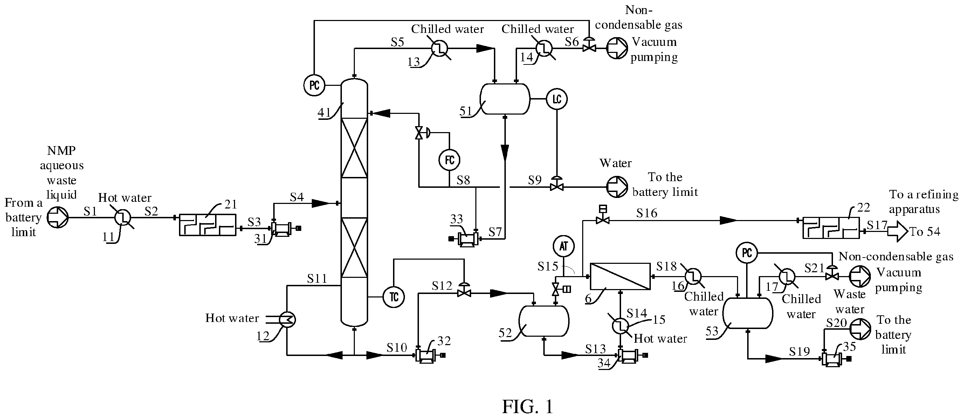

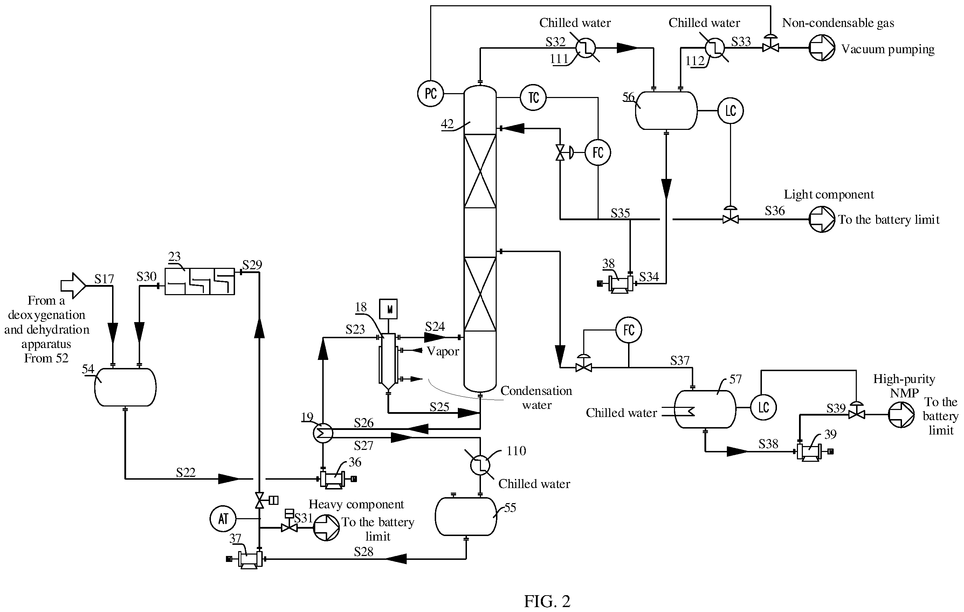

Refer to FIG. 1 to FIG. 4. Embodiment 1 of the present invention is as follows: Refer to FIG. 1 and FIG. 2. A high-purity NMP recovery device is provided, including a deoxygenation and dehydration apparatus and a refining apparatus. The deoxygenation and dehydration apparatus includes a deoxygenation preheater 11, a first deoxygenator 21, a dehydration tower 41, a dehydration tower-top condenser 13, a dehydration tower-top reflux tank 51, a molecular sieve membrane device raw material tank 52, a molecular sieve membrane pipe feed pump 34, a preheater 15 located before a molecular sieve membrane, a NaA molecular sieve membrane component 6, a second deoxygenator 22, and a dehydration tank 53 located after a molecular sieve membrane. The deoxygenation preheater 11 is connected to a raw material feed pipe and the first deoxygenator 21, and the dehydration tower 41 is connected to the first deoxygenator 21, the dehydration tower-top condenser 13, and the molecular sieve membrane device raw material tank 52. The dehydration tower-top reflux tank 51 is connected to the dehydration tower-top condenser 13 and the dehydration tower 41. The molecular sieve membrane pipe feed pump 34 is connected to the molecular sieve membrane device raw material tank 52 and the preheater 15 located before a molecular sieve membrane. The NaA molecular sieve membrane component 6 is connected to the preheater 15 located before a molecular sieve membrane, the molecular sieve membrane device raw material tank 52, and the second deoxygenator 22. Preferably, the NaA molecular sieve membrane component 6 is a liquid-phase dehydration molecular sieve membrane component. The deoxygenation and dehydration apparatus further includes a cooler 16 located after a molecular sieve membrane, the dehydration tank 53 located after a molecular sieve membrane, a vacuum condenser 17 located after a molecular sieve membrane, and a molecular sieve membrane pipe dehydration discharge pump 35. The cooler 16 located after a molecular sieve membrane is connected to the NaA molecular sieve membrane component 6 and the dehydration tank 53 located after a molecular sieve membrane. The dehydration tank 53 located after a molecular sieve membrane is connected to the vacuum condenser 17 located after a molecular sieve membrane and the molecular sieve membrane pipe dehydration discharge pump 35.

During operation of the high-purity NMP recovery device, the first deoxygenator 21 is first used for low-temperature deoxygenation, and then the dehydration tower 41 is used for low-temperature distillation for preliminary dehydration, to obtain an NMP aqueous solution with a concentration of 98%. Then, the NaA molecular sieve membrane component 6 is used for final dehydration (the NaA molecular sieve membrane component 6 has better selectivity for high-concentration NMP and a dehydration effect is better). If these steps are not performed in this sequence, for example, a NaA molecular sieve membrane is first used and then distillation is performed, the effect is not ideal. This causes low selectivity of the NaA molecular sieve membrane and high distillation temperature, which is not conducive to a reduction in oxidative decomposition of NMP. In addition, when low-temperature distillation for preliminary dehydration is performed to obtain the NMP aqueous solution with the concentration of 98%, a temperature of a tower kettle may be kept low, which is no more than 80 °C. If the concentration of NMP is excessively high, the temperature of the tower kettle is higher, or if the concentration of NMP is excessively low, the selectivity of the NaA molecular sieve membrane is reduced subsequently and a treatment amount is increased, resulting in an unsatisfactory purification effect.

Specifically, the deoxygenation preheater 11 performs heating by using hot water. A liquid inlet of the deoxygenation preheater 11 is connected to a first pipe S1 (that is, the foregoing raw material feed pipe), a liquid outlet 78 of the deoxygenation preheater 11 is connected to the first deoxygenator 21 through a second pipe S2, the first deoxygenator 21 is connected to a dehydration tower feed pump 31 through a third pipe S3, and the dehydration tower feed pump 31 is connected to a tower body middle section of the dehydration tower 41 through a fourth pipe S4. The top of the dehydration tower 41 is connected to the dehydration tower-top condenser 13 through a fifth pipe S5, and the dehydration tower-top reflux tank is connected to a dehydration tower-top vacuum condenser 14 through a sixth pipe S6. Non-condensable gas obtained after condensation by the dehydration tower-top vacuum condenser 14 is discharged through vacuum pumping. The dehydration tower-top reflux tank 51 is connected to a dehydration tower-top reflux discharge pump 33 through a seventh pipe S7, the dehydration tower-top reflux discharge pump 33 is connected to an upper part of the dehydration tower 41 through an eighth pipe S8, and the dehydration tower-top reflux discharge pump 33 discharges water through a ninth pipe S9. A tower kettle of the dehydration tower 41 is connected to a dehydration tower kettle discharge pump 32 through a tenth pipe S10, the tower kettle of the dehydration tower 41 is connected to an eleventh pipe S11, and the eleventh pipe S11 is connected to a dehydration tower thermosiphon reboiler 12. The dehydration tower kettle discharge pump 32 is connected to the molecular sieve membrane device raw material tank 52 through a twelfth pipe S12, the molecular sieve membrane device raw material tank 52 is connected to the molecular sieve membrane pipe feed pump 34 through a thirteenth pipe S13, and the molecular sieve membrane pipe feed pump 34 is connected to the NaA molecular sieve membrane component 6 through a fourteenth pipe S14. The fourteenth pipe S14 is connected to the preheater 15 located before a molecular sieve membrane, the NaA molecular sieve membrane component 6 is connected to the molecular sieve membrane device raw material tank 52 through a fifteenth pipe S15, and the NaA molecular sieve membrane component 6 is connected to the second deoxygenator 22 through a sixteenth pipe S16. The NaA molecular sieve membrane component 6 is connected to the dehydration tank 53 located after a molecular sieve membrane through an eighteenth pipe S18, and the eighteenth pipe S18 is connected to the cooler 16 located after a molecular sieve membrane. The dehydration tank 53 located after a molecular sieve membrane is connected to the molecular sieve membrane pipe dehydration discharge pump 35 through a nineteenth pipe S19, and the molecular sieve membrane pipe dehydration discharge pump 35 is connected to the outside through a twentieth pipe S20. The dehydration tank 53 located after a molecular sieve membrane is connected to the vacuum condenser 17 located after a molecular sieve membrane through a twenty-first pipe S21, and non-condensable gas obtained after condensation by the vacuum condenser 17 located after a molecular sieve membrane is discharged through vacuum pumping.

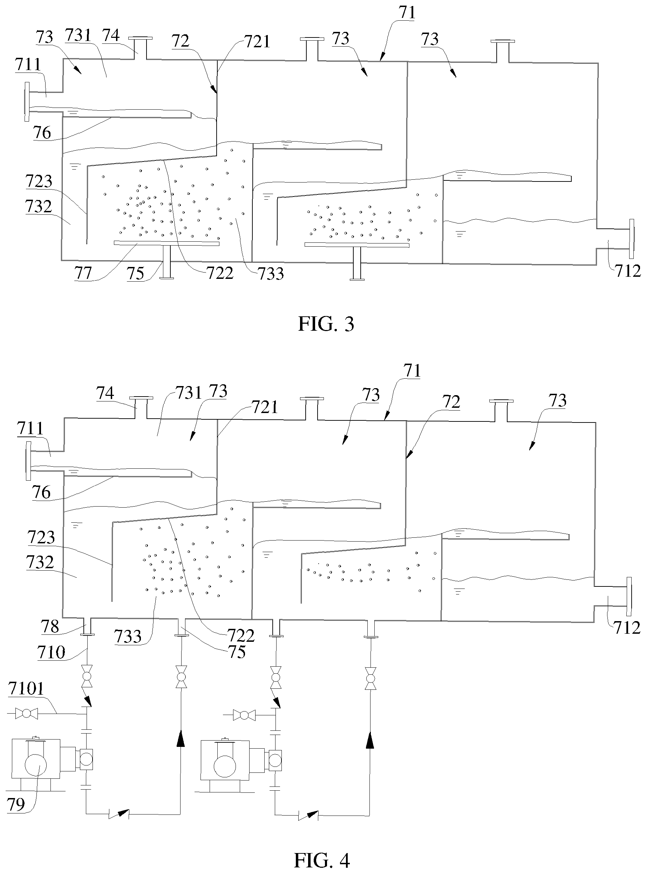

As shown in FIG. 3, to implement low-temperature deoxygenation, the first deoxygenator 21 includes a box 71. Several partition plates 72 are disposed in the box 71, and the partition plates 72 divide inner space of the box 71 into a plurality of chambers 73. Upper parts of the chambers 73 are gas-phase space 731, and lower parts of the chambers 73 are liquid-phase space. Lower parts of the partition plates 72 divide the liquid-phase space of the chambers 73 into first space 732 and second space 733 that are connected at the bottom. The second space 733 of the previous chamber 73 is connected to the gas-phase space 731 of the next chamber 73. Preferably, in the chamber 73, a volume of the first space 732 is smaller than a volume of the second space 733. The upper part of each of the chambers 73 has a vacuum pumping hole 74 that is connected to the corresponding gas-phase space 731, and the lower part of the chamber 73 other than the chamber 73 located at the end has a gas introducing opening 75 that is connected to the second space 733. The gas-phase space 731 of each of the chambers 73 is provided with an overflow weir 76, the box 71 is provided with a solvent inlet 711 and a solvent outlet 712, the overflow weir 76 of the chamber 73 located at the beginning is connected to the solvent inlet 711, and the second space 733 of the chamber 73 located at the end is connected to the solvent outlet 712.

It should be noted that the previous chamber 73 and the next chamber 73 described above are determined based on a sequence in which a solvent flows through the chambers, that is, the solvent enters the next chamber 73 from the second space 733 of the previous chamber 73. The chamber 73 located at the beginning described above is the chamber 73 that the solvent first enters when the solvent enters the first deoxygenator 21, and the chamber 73 located at the end described above is the chamber 73 in which the solvent finally stays before the solvent leaves the first deoxygenator 21.

In the two adjacent chambers 73, a height of the overflow weir 76 is related to pressure of the chamber 73 and a flow direction. Generally, under same pressure, the overflow weir 76 in the previous chamber 73 is disposed higher than the overflow weir 76 in the next chamber 73.

In some embodiments, there may be two chambers 73, that is, in this case, there is one partition plate 72. To ensure a better deoxygenation effect, there are preferably at least three chambers 73. In this case, there are two or more partition plates 72. For ease of description, in this embodiment, there are three chambers 73.

Preferably, the gas introducing opening 75 is located at the bottom of the chamber 73, so that inert gas introduced through the gas introducing opening 75 can better resolve dissolved oxygen in the solvent, which improves the deoxygenation effect. The inert gas includes but is not limited to nitrogen.

To further improve the deoxygenation effect, the second space 733 of at least some of the chambers 73 is provided with an aeration apparatus 77 that is connected to the gas introducing opening 75. The aeration apparatus 77 may be a diaphragm aerator. Diameters of openings of the aeration apparatus are 1 mm, an opening percentage is 10% to 15%, and bubbles generated are approximately 1 mm to 3 mm. A gas introducing amount is determined based on dissolved oxygen content of the liquid-phase and a treatment amount of the liquid-phase. The aeration apparatus 77 has advantages of a simple structure and low costs, and does not occupy external space of the first deoxygenator 21.

A depth of the overflow weir 76 is 3 mm to 6 mm. Because the overflow weir 76 is shallow, a pressure difference between an upper-layer solvent and a lower-layer solvent on the overflow weir 76 is extremely small, and impact of pressure on a bubble point of the solvent can be negligible. Because the solvent on the entire overflow weir 76 is close to the bubble point, gas solubility of the solvent is very small, and originally dissolved gas comes out, thereby implementing thermal deoxygenation.

A flow rate of the solvent on the overflow weir 76 or a length of the overflow weir 76 is controlled, so that a time in which a unit volume of solvent stays on the overflow weir 76 is greater than or equal to 10 seconds. This helps removal of the gas from the solvent.

A degassing temperature of the overflow weir 76 is 2 °C to 3 °C lower than the bubble point of the solvent flowing on the overflow weir. Specifically, due to a heat loss of the solvent, vacuum degrees (pressure) required by overflow weirs 76 in different chambers 73 are different. In two adjacent chambers 73, the vacuum degree of the gas-phase space 731 of the previous chamber 73 is lower than the vacuum degree of the gas-phase space 731 of the next chamber 73. During specific operation, the vacuum degree may be controlled based on a temperature of the overflow weir 76 detected online.

The partition plates 72 are located at an upper part of the box 71, which has two functions. One function is to separate two adjacent pieces of gas-phase space 731, so that degassing temperatures of the overflow weirs 76 in different chambers 73 each can be 2 °C to 3 °C lower than a bubble point of a solvent flowing into the corresponding chamber 73, to facilitate setting of different vacuum degrees for the gas-phase space 731 of different chambers 73. The other function is to separate gases and prevent gas back-mixing. Generally, oxygen content of gas in the gas-phase space 731 of the previous chamber 73 is higher than oxygen content of gas in the gas-phase space 731 of the next chamber 73. Separation of the gas-phase space 731 of the two adjacent chambers 73 helps maintain the deoxygenation effect. A function of the partition plates 72 located at a lower part of the box 71 is to separate the liquid-phase space to prevent the inert gas from escaping from the front of the first space 732.

In this embodiment, the partition plate 72 includes a first vertical section 721, an inclined section 722, and a second vertical section 723 that are connected in sequence from top to bottom. The first vertical section 721 is parallel to the second vertical section 723, and an angle between the inclined section 722 and the first vertical section 721 is greater than or equal to 90° and less than or equal to 110°. Preferably, the angle between the inclined section 722 and the first vertical section 721 is 95°, which facilitates upward discharge of the inert gas and avoids impact of gas accumulation on the vacuum degree.

The following briefly describes an operating principle of the first deoxygenator 21 by using an example in which the first deoxygenator 21 performs deoxygenation on NMP waste liquid with water content of 85% in an NMP recovery procedure.

The NMP waste liquid to be deoxygenated enters the overflow weir 76 of the chamber 73 located at the beginning from the solvent inlet 711, and a vacuum degree of the gas-phase space 731 of the chamber 73 located at the beginning is controlled by using the vacuum pumping hole 74. A temperature of the NMP waste liquid on the overflow weir 76 is made to be 2 °C to 3 °C lower than a solvent bubble point under this vacuum degree. Because the overflow weir 76 is shallow, a pressure difference between upper and lower sides is extremely small, and impact of pressure on the bubble point can be negligible. Because the NMP waste liquid on the entire overflow weir 76 is close to the bubble point, gas solubility of the NMP waste liquid is very small, and originally dissolved gas comes out. The degassed NMP waste liquid overflows the overflow weir 76, and flows downward into the liquid-phase space below. The NMP waste liquid crosses the partition plate 72 in the liquid-phase space, and flows from the first space 732 into the second space 733. The aeration apparatus 77 in the second space 733 introduces nitrogen into the second space 733. Because there is a specific liquid height in the second space 733, pressure generated due to a liquid level difference increases the gas solubility of the NMP waste liquid located at a lower part of the second space 733, and a part of the nitrogen is dissolved in the NMP waste liquid located at the lower part of the second space 733. As the NMP waste liquid rises in the second space 733, the pressure gradually decreases, the nitrogen dissolved in the NMP waste liquid reverts to a gas phase and escapes, and dissolved oxygen in the NMP waste liquid is brought out. Then, the rising NMP waste liquid enters the overflow weir 76 of the next chamber 73. The dissolved gas is removed, and an entire procedure of degassing, nitrogen filling, and removal is completed. Subsequently, next round of degassing, nitrogen filling, and removal is performed. So far, a concentration of the dissolved oxygen in the NMP waste liquid after deoxygenation has reached a concentration required by the process, which provides a good condition for suppressing side reactions in subsequent distillation.

As shown in FIG. 4, in some embodiments, the first deoxygenator 21 further includes a gas-liquid mixing pump 79. The gas-liquid mixing pump 79 replaces the foregoing diaphragm aerator (the aeration apparatus 77). In this case, the lower part of the chamber 73 other than the chamber 73 located at the end has a liquid outlet 78 that is connected to the first space 732, an output port of the gas-liquid mixing pump 79 is connected to the gas introducing opening 75, and an inlet of the gas-liquid mixing pump 79 is connected to the liquid outlet 78.

Specifically, the gas-liquid mixing pump 79 is connected to the liquid outlet 78 through a main pipe 710, the main pipe 710 is connected to an inert gas access pipe 7101, and the inert gas access pipe 7101 is configured to introduce inert gas into the main pipe 710. In other words, in the first deoxygenator in this embodiment, the inert gas is not directly introduced into the second space 733 of the chamber 73 through the gas introducing opening 75 of the chamber 73, but is introduced into the second space 733 after being mixed with a part of the solvent.

The solvent in the first space 732 and the inert gas are mixed and sucked into the gas-liquid mixing pump 79. A high-speed rotating pump impeller of the gas-liquid mixing pump 79 mixes and stirs the solvent and the inert gas. The inert gas is fully dissolved in the solvent due to the pressurized mixing in the pump. When the solvent returns to the second space 733, as a liquid height increases and pressure decreases, the inert gas dissolved in the solvent comes out in a form of bubbles of 20 µm to 100 µm. In this way, a specific surface area of a unit volume of inert gas is larger, utilization degree of the inert gas is higher, and produced exhaust gas is less, which can reduce a load of a vacuum device.

The refining apparatus includes a refining tower raw material tank 54, a refining tower feed pump 36, a refining tower scraper falling-film evaporation reboiler 18, a refining tower 42, a refining tower kettle discharge cooler 110, a refining tower kettle liquid temporary storage tank 55, a refining tower return and tower kettle discharge pump 37, and a third deoxygenator 23. The refining tower raw material tank 54 is connected to the second deoxygenator 22 and the refining tower feed pump 36. The refining tower scraper falling-film evaporation reboiler 18 is connected to a lower part of the refining tower 42 and the refining tower feed pump 36. The refining tower kettle discharge cooler 110 is connected to the bottom of the refining tower 42 and the refining tower kettle liquid temporary storage tank 55. The refining tower kettle liquid temporary storage tank 55 is connected to the refining tower return and tower kettle discharge pump 37, the refining tower return and tower kettle discharge pump 37 is connected to the outside and the third deoxygenator 23, and the third deoxygenator 23 is connected to the refining tower raw material tank 54. The refining tower 42 has a side discharge port. The refining apparatus further includes an NMP product cooling tank 57, and the NMP product cooling tank 57 is connected to the side discharge port.

To be specific, the second deoxygenator 22 is connected to the refining tower raw material tank 54 through a seventeenth pipe S17, the refining tower raw material tank 54 is connected to the refining tower feed pump 36 through a twenty-second pipe S22, and the refining tower feed pump 36 is connected to the refining tower scraper falling-film evaporation reboiler 18 through a twenty-third pipe S23. The twenty-third pipe S23 is connected to a refining tower feed-before preheater 19. The refining tower scraper falling-film evaporation reboiler 18 is connected to the lower part of the refining tower 42 through a twenty-fourth pipe S24. A twenty-fifth pipe S25 at the bottom of the refining tower scraper falling-film evaporation reboiler 18 intersects with a tower kettle discharge pipe of the refining tower 42 to form a twenty-sixth pipe S26, and the twenty-sixth pipe S26 is connected to the refining tower feed-before preheater 19. The refining tower feed-before preheater 19 is connected to the refining tower kettle liquid temporary storage tank 55 through a twenty-seventh pipe S27, and the twenty-seventh pipe S27 is connected to the refining tower kettle discharge cooler 110. The refining tower kettle liquid temporary storage tank 55 is connected to the refining tower return and tower kettle discharge pump 37 through a twenty-eighth pipe S28. One passage of the refining tower return and tower kettle discharge pump 37 is connected to the third deoxygenator 23 through a twenty-ninth pipe S29, the other passage is connected to the outside through a thirty-first pipe S31, and a heavy component is discharged to the outside through the thirty-first pipe S31. The third deoxygenator 23 is connected to the refining tower raw material tank 54 through a thirtieth pipe S30. The top of the refining tower 42 is connected to a refining tower-top reflux tank 56 through a thirty-second pipe S32, and the thirty-second pipe S32 is connected to a refining tower-top condenser 111. The refining tower-top reflux tank 56 is connected to a refining tower-top vacuum condenser 112 through a thirty-third pipe S33, and non-condensable gas obtained after condensation by the refining tower-top vacuum condenser 112 is discharged through vacuum pumping. The refining tower-top reflux tank 56 is connected to a refining tower-top reflux discharge pump 38 through a thirty-fourth pipe S34. The refining tower-top reflux discharge pump 38 is connected to an upper part of the refining tower 42 through a thirty-fifth pipe S35, and the refining tower-top reflux discharge pump 38 is connected to the outside through a thirty-sixth pipe S36 to discharge a light component. The side discharge port of the refining tower 42 is connected to the NMP product cooling tank 57 through a thirty-seventh pipe S37, the NMP product cooling tank 57 is connected to an NMP product discharge pump 39 through a thirty-eighth pipe S38, and the NMP product discharge pump 39 is connected to the outside through a thirty-ninth pipe S39 to discharge a high-purity NMP product.

This embodiment further provides a high-purity NMP recovery process that is based on the foregoing high-purity NMP recovery device.

The following briefly describes a procedure by using an example.

An NMP aqueous solution with water content of 80% enters the deoxygenation preheater 11 through the first pipe S1 (in a lithium battery coating machine process, an NMP aqueous solution with water content of 80% to 90% is common, but the specific water content of the NMP aqueous solution is not limited during actual operation). A temperature of the NMP aqueous solution increases to 55 °C preliminarily, and the NMP aqueous solution enters the first deoxygenator 21. After deoxygenation and degassing by the first deoxygenator 21, the temperature of the NMP aqueous solution is approximately 50 °C, and the NMP aqueous solution enters the dehydration tower 41 through the dehydration tower feed pump 31.

When the water content of the NMP aqueous solution is higher than 5% and the temperature exceeds 80 °C, a rate of oxidative decomposition of NMP increases. Although the NMP aqueous solution is deoxygenated before entering the dehydration tower 41, it cannot be fully ensured that outside air does not enter the system, because the dehydration tower 41 operates under negative pressure. To ensure product quality, process conditions of the dehydration tower 41 are as follows: Pressure at the top of the tower is controlled to be 4 kPa, a wire mesh filler is used, pressure drop of the entire tower is approximately 0.8 kPa, a temperature of the tower kettle is controlled to be 77.5 °C, and a temperature of the top of the tower is 28 °C. A discharge material from the top of the tower is pure water, and the pure water is condensed by the dehydration tower-top condenser 13 and then enters the dehydration tower-top reflux tank 51. Then, a part of the pure water returns to a distillation tower as reflux, and a part of the pure water is sent out of a battery limit to be reused as an absorbent for upstream NMP. A material that enters the molecular sieve membrane device raw material tank 52 through the twelfth pipe S12 is an NMP aqueous solution with a concentration of 98%.

The NaA molecular sieve membrane component 6 uses a liquid-phase dehydration molecular sieve membrane, and an advantage is as follows: A NaA molecular sieve membrane liquid-phase dehydration process shows excellent osmotic dehydration performance in an NMP aqueous solution with an extremely low water concentration (according to the literature (a master's thesis "The research on pervaporative applications, fouling and regeneration of NaA zeolite membrane", which is written by Zeng Wenhao), a separation coefficient of water/NMP at 100 °C is 10013 when water content of an NMP solution is 0.1%; when the water content of the NMP solution is 2%, the separation coefficient of water/NMP at 80 °C is 1389, and the separation coefficient of water/NMP at 100 °C is 780; and the separation coefficient of water/NMP at 80 °C is 134 when the water content of the NMP solution is 20%). The NMP aqueous solution with the concentration of 98% enters the preheater 15 located before a molecular sieve membrane. After heat exchange, a temperature of the NMP aqueous solution is 80 °C. Then, the NMP aqueous solution returns to the molecular sieve membrane device raw material tank 52 through the NaA molecular sieve membrane component 6. The cycle is repeated. It should be noted that as water of a fluid flowing through the fifteenth pipe S15 is gradually extracted (it is difficult for NMP but easier for water to pass through the NaA molecular sieve membrane, and the concentration of NMP is increasingly high with time going by), a heat exchange temperature of the preheater 15 located before a molecular sieve membrane can be gradually increased to increase a dehydration rate (a higher temperature indicates a higher dehydration rate). After final dehydration, water content of liquid flowing through the fifteenth pipe S15 is less than 100 ppm and a temperature is 95 °C. After inspection is passed, the sixteenth pipe S16 is opened, a deoxygenated material enters the refining apparatus, and a temperature is approximately 90 °C.

A material flowing through the eighteenth pipe S18 is water containing an extremely small quantity of NMP (generally, NMP content is controlled to be 200 ppm). After being condensed by the cooler 16 located after a molecular sieve membrane and the vacuum condenser 17 located after a molecular sieve membrane, the material is sent out of the battery limit through the molecular sieve membrane pipe dehydration discharge pump 35 to be reused as an absorbent for upstream NMP.

A material from the second deoxygenator 22 is temporarily stored in the refining tower raw material tank 54, and then sent to the refining tower scraper falling-film evaporation reboiler 18 through the refining tower feed pump 36. A heating steam jacket is disposed on the outside of a housing of the scraper falling-film evaporation reboiler 18, and a rotatable scraper is disposed on the inside of the housing. The scraper is driven by a rotating shaft at a center of a cylinder. After being added tangentially from an upper part of the evaporator, the raw material liquid is driven by gravity and the rotating scraper, and forms a down-spin thin film along an inner wall of the housing. Finished liquid is discharged from the bottom of the evaporator, and secondary steam passes through a demister and then is discharged from the upper part. The scraper falling-film evaporation reboiler has several advantages: It can treat some materials containing solids. Exhaust gas of a coating machine contains trace quantities of soluble lithium salts and insoluble particulate matter, and materials containing particulate matter can be concentrated and then discharged. A heat transfer coefficient of the scraper falling-film evaporation reboiler is high, and a total heat transfer coefficient is 1750 to 7000 W/(m²·°C), while heat transfer coefficients of thermosiphon and kettle reboilers are lower than 1000 W/(m²·°C). A time in which the material stays in the scraper falling-film evaporation reboiler is short, which is generally 5 seconds to 10 seconds. The oxidative decomposition of NMP depends on a time in which NMP stays in a high-temperature state, and a shorter staying time indicates a lower degree of the oxidation decomposition. Therefore, using the scraper falling-film evaporation reboiler can reduce a probability of an NMP side reaction, which reduces the oxidative decomposition of NMP to a greater extent and increases a recovered and purified amount of NMP. When another type of reboiler is used, a staying time of NMP in a recovery process exceeds 10 seconds, and a probability of an NMP side reaction is high.

A vaporization ratio of the material entering the scraper falling-film evaporation reboiler 18 is approximately 10% to 50%, and a temperature is approximately 120 °C. The vaporized material enters the lower part of the refining tower 42. The unvaporized material and a material discharged from the tower kettle enter the twenty-sixth pipe S26, then enter the refining tower feed-before preheater 19 for heat exchange to cool down to 100 °C, and are further cooled by the refining tower kettle discharge cooler 110 to 90 °C and temporarily stored in the refining tower kettle liquid temporary storage tank 55. The unvaporized material at an outlet of the refining tower scraper falling-film evaporation reboiler 18 is cooled to 90 °C, a temperature of the entire tower kettle does not exceed 105 °C, a staying time in the cooling procedure is controlled to be approximately 10 seconds to 20 seconds, and a total staying time at high temperature is controlled to be within 30 seconds. This reduces the oxidative decomposition of NMP to a greater extent. In an existing process, temperatures of the outlet of the reboiler and the tower kettle are generally higher than 120 °C, and there are no cooling measures. The staying time is far more than 30 seconds, and a probability of a side reaction is high.

The material temporarily stored in the refining tower kettle liquid temporary storage tank 55 is transferred by the refining tower return and tower kettle discharge pump 37 to the third deoxygenator 23 for deoxygenation. The deoxygenated material returns to the refining tower raw material tank 54. At this time, a temperature of the material is approximately 85 °C. A reason for such a cycle is as follows: The vaporization ratio of the material passing through the reboiler only once is not high, and a large quantity of liquid materials are not vaporized. This procedure needs to be repeated, and a distillation procedure can be terminated only when content of a heavy component and particulate matter in the material in the refining tower kettle liquid temporary storage tank 55 is approximately 50%. A heavy component waste material produced during distillation needs to be cooled, and sent out of the battery limit through the thirty-first pipe S31 for other treatment.

Process conditions of the refining tower 42 are as follows: Pressure at the top of the tower is controlled to be 2 kPa, a wire mesh filler is used, pressure drop of the entire tower is approximately 2 kPa, a temperature of the tower kettle is controlled to be 105 °C, and a temperature of the top of the tower is 40 °C to 80 °C. Materials discharged from the top of the tower are mainly a small quantity of water and light components contained by NMP waste liquid. The materials are condensed by the refining tower-top condenser 111 and then enter the refining tower-top reflux tank 56. Some of the materials return to the refining tower 42 as reflux, and some light components are sent out of the battery limit for other treatment. A material discharged from the side discharge port is high-purity NMP. The material is sent to the NMP product cooling tank 57 for temporary storage and cooling, and then is transferred by the NMP product discharge pump 39 to the battery limit to obtain a product.

The following describes outstanding advantages of the high-purity NMP recovery device.
1. At present, in an industrial production procedure, a temperature for NMP recovery is generally higher than 100 °C, and a product obtained after vacuum distillation still contains a small quantity of N-Methylsuccinimide (NMS) and N-Methylacetamide. This cannot satisfy a requirement of high-purity NMP. The high-purity NMP recovery device can perform low-temperature dehydration and deoxygenation (deoxygenation in the deoxygenator at a temperature of 50 °C to 55 °C, dehydration in the dehydration tower at a temperature lower than 80 °C, and dehydration in the NaA molecular sieve membrane component at a temperature of 80 °C to 95 °C) on NMP waste liquid, and reduce a staying time during high-temperature refining (a staying time is approximately 5 seconds to 10 seconds when the scraper falling-film evaporation reboiler is used, an unvaporized material at the outlet of the reboiler is cooled to 90 °C, a staying time in the cooling procedure is controlled to be approximately 10 seconds to 20 seconds, and a total staying time is controlled to be within 30 seconds), so as to reduce oxidative decomposition of NMP and obtain high-purity electronic-grade NMP.
2. The NaA molecular sieve membrane component 6 is a liquid-phase dehydration molecular sieve membrane component, and a frequency of maintenance and cleaning is low, which greatly prolongs a service life and maintenance cycle of NaA and improves production efficiency. In addition, energy consumption is low. NaA molecular sieve membrane liquid-phase dehydration does not require vaporization, which is energy-saving.
3. For some materials that contain only water or production of an industrial-grade NMP product, when the high-purity NMP recovery device is used, only the deoxygenation and dehydration apparatus in the high-purity NMP recovery device is needed without using the refining apparatus, and flexibility of use is high.

The foregoing embodiments are merely optional embodiments of the present invention, and are not intended to limit the patent scope of the present invention. Under the inventive concept of the present invention, any equivalent structural variation or direct/indirect application in other related technical fields made based on the content of the specification and the accompanying drawings of the present invention shall fall within the patent protection scope of the present invention.

## Claims

1. A high-purity NMP recovery device, comprising a deoxygenation and dehydration apparatus, wherein the deoxygenation and dehydration apparatus comprises a deoxygenation preheater, a first deoxygenator, a dehydration tower, a dehydration tower-top condenser, a dehydration tower-top reflux tank, a molecular sieve membrane device raw material tank, a molecular sieve membrane pipe feed pump, a preheater located before a molecular sieve membrane, a NaA molecular sieve membrane component, a second deoxygenator, and a dehydration tank located after a molecular sieve membrane; the deoxygenation preheater is connected to a raw material feed pipe and the first deoxygenator, and the dehydration tower is connected to the first deoxygenator, the dehydration tower-top condenser, and the molecular sieve membrane device raw material tank; the dehydration tower-top reflux tank is connected to the dehydration tower-top condenser and the dehydration tower; the molecular sieve membrane pipe feed pump is connected to the molecular sieve membrane device raw material tank and the preheater located before a molecular sieve membrane; and the NaA molecular sieve membrane component is connected to the preheater located before a molecular sieve membrane, the molecular sieve membrane device raw material tank, and the second deoxygenator.

2. The high-purity NMP recovery device according to claim 1, wherein the first deoxygenator comprises a box, several partition plates are disposed in the box, the partition plates divide inner space of the box into a plurality of chambers, upper parts of the chambers are gas-phase space, lower parts of the chambers are liquid-phase space, lower parts of the partition plates divide the liquid-phase space of the chambers into first space and second space that are connected at the bottom, and the second space of the previous chamber is connected to the gas-phase space of the next chamber; and the upper part of each of the chambers has a vacuum pumping hole that is connected to the corresponding gas-phase space, the lower part of the chamber other than the chamber located at the end has a gas introducing opening that is connected to the second space, the gas-phase space of each of the chambers is provided with an overflow weir, the box is provided with a solvent inlet and a solvent outlet, the overflow weir of the chamber located at the beginning is connected to the solvent inlet, and the second space of the chamber located at the end is connected to the solvent outlet.

3. The high-purity NMP recovery device according to claim 2, wherein a depth of the overflow weir in the first deoxygenator is 3 mm to 6 mm.

4. The high-purity NMP recovery device according to claim 2, wherein the second space of at least some of the chambers in the first deoxygenator is provided with an aeration apparatus that is connected to the gas introducing opening.

5. The high-purity NMP recovery device according to claim 2, wherein the first deoxygenator further comprises a gas-liquid mixing pump, the lower part of the chamber other than the chamber located at the end has a liquid outlet that is connected to the first space, an output port of the gas-liquid mixing pump is connected to the gas introducing opening, and an inlet of the gas-liquid mixing pump is connected to the liquid outlet.

6. The high-purity NMP recovery device according to claim 2, wherein a degassing temperature of the overflow weir in the first deoxygenator is 2 °C to 3 °C lower than a bubble point of a solvent flowing on the overflow weir.

7. The high-purity NMP recovery device according to claim 2, wherein the partition plate comprises a first vertical section, an inclined section, and a second vertical section that are connected in sequence from top to bottom, the first vertical section is parallel to the second vertical section, and an angle between the inclined section and the first vertical section is greater than or equal to 90° and less than or equal to 110°.

8. The high-purity NMP recovery device according to claim 1, further comprising a refining apparatus, wherein the refining apparatus comprises a refining tower raw material tank, a refining tower feed pump, a refining tower scraper falling-film evaporation reboiler, a refining tower, a refining tower kettle discharge cooler, a refining tower kettle liquid temporary storage tank, a refining tower return and tower kettle discharge pump, and a third deoxygenator; the refining tower raw material tank is connected to the second deoxygenator and the refining tower feed pump; the refining tower scraper falling-film evaporation reboiler is connected to a lower part of the refining tower and the refining tower feed pump; the refining tower kettle discharge cooler is connected to the bottom of the refining tower and the refining tower kettle liquid temporary storage tank; the refining tower kettle liquid temporary storage tank is connected to the refining tower return and tower kettle discharge pump, the refining tower return and tower kettle discharge pump is connected to the outside and the third deoxygenator, and the third deoxygenator is connected to the refining tower raw material tank; and the refining tower has a side discharge port.

9. The high-purity NMP recovery device according to claim 8, wherein the refining apparatus further comprises an NMP product cooling tank, and the NMP product cooling tank is connected to the side discharge port.

10. A high-purity NMP recovery process, wherein the process is based on the high-purity NMP recovery device according to any one of claims 1 to 9.
